# EUROPEAN PATENT APPLICATION

(11) **EP 0 800 814 A2**
(43) Date of publication of application: **15.10.1997**
(21) Application number: 97301724.7
(22) Date of filing: 14.03.1997
(51) Int. Cl.: A61K 7/06

(54) **Antimicrobial hair treatment composition**

(30) Priority: 12.04.1996 GB 9607568
(71) Applicant: UNILEVER PLC, London EC4P 4BQ (GB); UNILEVER N.V., 3013 AL Rotterdam (NL)
(72) Inventor: Eccleson, Graham Charles, Bebington, Wirral, Merseyside L63 3JW (GB); Hague, Jonathan David, Bebington, Wirral, Merseyside L63 3JW (GB); Konidaris, Peter Christopher, Bebington, Wirral, Merseyside L63 3JW (GB)
(74) Representative: Rots, Maria Johanna Francisca

(57) **Abstract**

The present invention relates to antimicrobial hair treatment composition which is a shampoo composition comprising an antimicrobial compound selected from certain 1-hydroxy-6-substituted pyridine-2-one and 2-thione compounds, a shampoo surfactant and a polymeric, water-soluble cationic deposition aid. The pH of the hair treatment composition is adjusted to less than 7, so that the antimicrobial compound is present as insoluble particles in the hair treatment composition. In this way, enhanced antidandruff activity can be obtained from a rinse-off shampoo composition by utilising the antimicrobial compound in insoluble form and in combination with a cationic deposition polymer as a deposition aid for the insoluble particles of antimicrobial compound.

## Description

### FIELD OF THE INVENTION

This invention relates to a hair treatment composition, and particularly to a hair treatment composition which is a shampoo composition containing an antimicrobial agent.

### Background and Prior Art

Insoluble particulate metal pyrithiones are acknowledged as antimicrobial agents which can be incorporated into antimicrobial compositions, such as antidandruff hair shampoos. The zinc salt (hereinafter referred to as ZnPTO) is widely used in this context. Generally, dispersed particles of the ZnPTO are suspended in the composition, which is then applied to the hair to deposit the ZnPTO on the hair and scalp.

A problem encountered with such compositions is that it is difficult to obtain a stable dispersion containing ZnPTO, since its density can lead to separation during storage. Steps taken to prevent separation of the ZnPTO particles have hitherto included restriction of the formulation base to highly viscous emulsions or gels, e.g., by incorporation of thickeners or bulking agents, such as clays or pearlescers, to give structural viscosity to the system and prevent ZnPTO settlement. Such an approach is frequently impractical for shampoo compositions, and can give cloudy, aesthetically-inferior products.

It is also known that certain 1-hydroxy-2-pyridone derivatives are effective as antimicrobial agents with specific applicability to dandruff control. 1-hydroxy-4-methyl-6-(2,4,4-trimethylpentyl)-2-pyridone has been described in US 4,185,106 and US 4,711,775 as exhibiting good antimicrobial efficacy and solubility in shampoo formulations. The compound is, however, expensive to prepare.

Furthermore, a problem encountered with soluble actives is that when incorporated in dissolved form into hair treatment compositions, effective deposition of the active from the composition onto the hair is difficult to obtain, so the active tends to be washed away during rinsing of the composition from the hair, leading to reduced antidandruff efficacy.

WO95/11233 (Olin Corporation) describes a specific class of 1-hydroxy-6-substituted pyridine-2-ones and 2-thiones, a process for their preparation and their use as antimicrobials in industrial and cosmetic applications generally, ranging from soaps and shampoos to sealants and other polymer compositions, most especially paints. According to WO95/11233, an advantage of these compounds is their solubility in water and organic solvents.

We have now found that the compounds of WO/9511233 can be formulated as insoluble agents in a hair treatment composition, by adjusting the pH of the composition to below 7, and that enhanced deposition onto the hair and scalp of these compounds, and therefore enhanced antidandruff activity can be obtained from a rinse-off shampoo composition by utilising the compounds in insoluble form and in combination with a cationic deposition polymer as a deposition aid for the insoluble particles of antimicrobial compound.

### SUMMARY OF THE INVENTION

The present invention provides an antimicrobial hair treatment composition which is a shampoo composition comprising the following components:
(a) an antimicrobial compound of the formula: 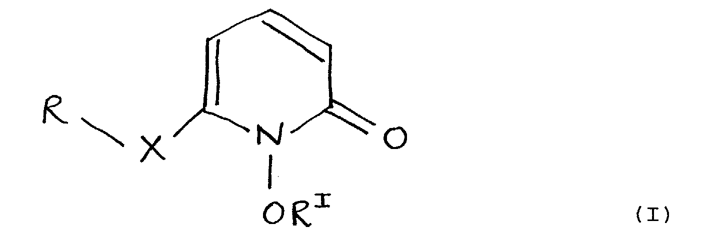 wherein X is an oxygen or sulphur moiety and R is a substituted or unsubstituted hydrocarbon radical having between 1 and 20 carbon atoms, with the proviso that R is other than chlorobenzyl, and R^{I} is hydrogen or a cation selected from the group consisting of alkali metals, alkaline earth metals, ammonium and organic ammonium ions, or a compound of the formula: 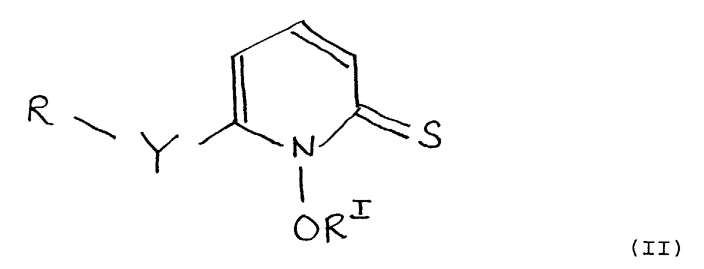 wherein Y is an oxygen, sulphur, or NR^{II} moiety wherein N is nitrogen and R and R^{II} are independently each a substituted or unsubstituted hydrocarbon radical having between 1 and 20 carbon atoms, or a compound of the formula: 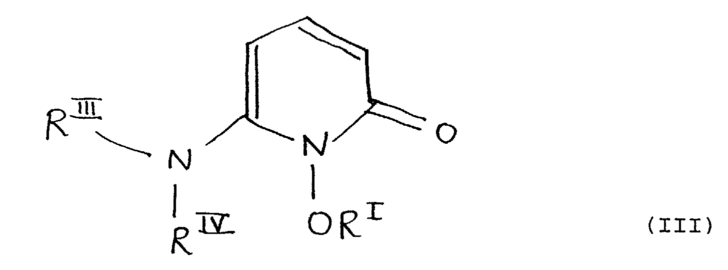 wherein R^{III} and R^{IV} are independently either hydrogen or a substituted or unsubstituted hydrocarbon radicals having between 1 and 20 carbon atoms, with the proviso that either R or R^{II} is a hydrocarbon radical;
(b) at least one shampoo surfactant; and
(c) a polymeric, water-soluble cationic deposition aid;
in which the pH of the hair treatment composition is less than 7, so that the antimicrobial compound is present as insoluble particles in the hair treatment composition.

### Detailed Description and Preferred Embodiments

Preferred hydrocarbon radicals for the R group in the above formulae (I), (II), and (III) are aliphatic hydrocarbons having between 3 and 15 carbons, more preferably a straight chain hydrocarbon having between 5 and 10 carbons. The term "substituted hydrocarbon" is intended to include substituents such as halogen, chloro, iodo, fluoro or bromo, alkoxy such as methoxy, ethoxy, propoxy or butoxy, nitro, thio, amino combinations thereof, and the like. Illustrative hydrocarbon groups include n-octyl, 2,4,4-trimethylpentyl, 3,5,5-trimethylhexyl, combinations thereof, and the like.

Examples of R^{I} include sodium, lithium, potassium, calcium, magnesium, ammonium and mono-, di-, or tri-ethanolamine.

A preferred antimicrobial compound in accordance with the invention is the monoethanolamine salt of the compound of formula (I) where R = octyl and X = oxygen, (available commercially from Olin Corporation under the designation HP101).

The amount of antimicrobial compound incorporated into the compositions of the invention may depend on the type of composition and the exact nature of the material used. A preferred amount of antimicrobial compound is from about 0.001 to about 5% by weight of the total composition, more preferably from about 0.1 to about 3% by weight.

The composition according to the invention comprises at least one shampoo surfactant.

Suitable shampoo surfactants are selected from anionic, nonionic, amphoteric and zwitterionic surfactants, and mixtures thereof.

Suitable anionic surfactants include the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from one to 10 ethylene oxide or propylene oxide units per molecule, and preferably contain 2 to 3 ethylene oxide units per molecule.

Examples of suitable anionic surfactants include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauroyl isethionate and sodium N-lauryl sarcosinate. The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

Nonionic surfactants suitable for use in composition of the invention may include condensation products of aliphatic (C₈-C₁₈) primary or secondary linear or branched chain alcohols or phenols with alkylene oxides, usually ethylene oxide and generally having from 6 to 30 ethylene oxide group. Other suitable nonionics include mono- or di-alkyl alkanolamides. Examples include coco mono- or di-ethanolamide and coco monoisopropanolamide.

Amphoteric and zwitterionic surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates alkyl amidopropyl hydroxysultaines, acyl taurates and acyl glutamates, wherein the alkyl and acyl groups have from 8 to 19 carbon atoms. Examples include lauryl amine oxide, cocodimethyl sulphopropyl betaine and preferably lauryl betaine, cocamidopropyl betaine and sodium cocamphopropionate.

The surfactants are present in shampoo compositions of the invention in an amount of from 0.1 to 50% by weight, preferably from 0.5 to 30% by weight.

Hair treatment compositions of the invention may also contain one or more conditioning agents, as are well known in the art. The conditioning agents may include cationic surfactants, silicones, protein hydrolysate, quaternised protein hydrolysate, and other materials which are known in the art as having desirable hair conditioning properties.

### Examples of cationic surfactants include:

quaternary ammonium hydroxides, e.g., tetramethylammonium hydroxide, alkyltrimethylammonium hydroxides wherein the alkyl group has from about 8 to 22 carbon atoms, for example octyltrimethylammonium hydroxide, dodecyltrimethylammonium hydroxide, hexadecyltrimethylammonium hydroxide, cetyltrimethylammonium hydroxide, octyldimethylbenzylammonium hydroxide, decyldimethylbenzylammonium hydroxide, stearyldimethylbenzylammonium hydroxide, didodecyldimethylammonium hydroxide, dioctadecyldimethylammonium hydroxide, tallow trimethylammonium hydroxide, cocotrimethylammonium hydroxide, and the corresponding salts thereof, e.g., chlorides

Cetylpyridinium hydroxide or salts thereof, e.g., chloride
Quaternium -5
Quaternium -31
Quaternium -18
and mixtures thereof.

In hair treatment compositions according to the invention, the level of cationic surfactant is preferably from 0.01 to 10%, more preferably 0.05 to 5%, most preferably 0.1 to 2% by weight of the composition.

Silicones are the most preferred conditioning agents.

Suitable silicones include volatile and non-volatile silicones, such as for example polyalkylsiloxanes, polyalkylaryl siloxanes, siloxane gums and resins, cyclomethicones, aminofunctional silicones, quaternary silicones and mixtures thereof. Silicone oil is a particularly preferred conditioning agent for hair. The silicone may be in the form of a low viscosity oil which may contain a high viscosity oil or gum in solution. Alternatively, the high viscosity material may be in the form of an emulsion in water. The emulsion may be of high viscosity oil or of a solution of gum in a lower viscosity oil. The particle size of the oil phase may be anywhere in the range from 30 nanometres to up to 200 microns average size.

The silicone oil may suitably be a polydimethylsiloxane with an average particle size of less than 20 microns and preferably less than 2 microns. Small particle size enables a more uniform distribution of silicone conditioning agent for the same concentration of silicone in the composition. Advantageously, a silicone with a viscosity in the range 1-20 million cst is used. The silicone is preferably emulsion-polymerised, since this enables silicones of very high viscosity to be more easily processed. The silicone can be cross-linked.

Suitable protein hydrolysate include lauryl dimonium hydroxy propylamino hydrolysed animal protein, available commercially under the trade name LAMEQUAT L, and hydrolysed keratin containing sulphur-bearing amino acids, available commercially under the trade name CROQUAT WKP.

In accordance with the invention, the hair treatment composition contains a polymeric water-soluble deposition aid for the insoluble particles of antimicrobial compound. By "deposition aid" is meant an agent which enhances deposition of the insoluble particles of antimicrobial compound on the intended site, i.e., the hair and/or the scalp.

The deposition aid will generally be present at levels of from 0.01 to 5%, preferably from about 0.5 to 1%, more preferably from about 0.08% to about 0.5% by weight. The deposition aid may be a homopolymer or be formed from two or more types of monomers. The molecular weight of the polymer will generally be between 5 000 and 20 000 000, typically at least 10 000 and preferably in the range 100 000 to about 10 000 000. The polymers will have cationic nitrogen containing groups such as quaternary ammonium or protonated amino groups, or a mixture thereof.

The cationic charge density of the deposition aid, which is defined as the reciprocal of the molecular weight of a monomeric unit of the polymer containing 1 charge, has been found to need to be at least 0.1 meq/g, preferably above 0.8 or higher. The cationic charge density should not exceed 4 meq/g, it is preferably less than 3 and more preferably less than 2 meq/g. The charge density can be measured using conductimetric analysis and should be within the above limits at the desired pH of use, which will in general be below 7 and preferably between 4 and 6.

The cationic nitrogen-containing group will generally be present as a substituent on a fraction of the total monomer units of the deposition aid. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic deposition aids include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

Suitable cationic amino and quaternary ammonium monomers include, for example, vinyl compounds substituted with dialkyl aminoalkyl acrylate, dialkylamino alkylmethacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidine, e.g., alkyl vinyl imidazolium, alkyl vinyl pyridinium, and alkyl vinyl pyrrolidine salts. The alkyl portions of these ,monomers are preferably lower alkyls such as the C₁-C₃ alkyls, more preferably C₁ and C₂ alkyls.

Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C₁-C₇ hydrocarbyls, more preferably C₁-C₃, alkyls.

The deposition aid can comprise mixtures of monomer units derived from amine- and/or quaternary ammonium-substituted monomer and/or compatible spacer monomers.

Suitable deposition aids include, for example: cationic copolymers of 1-vinyl-2-pyrrolidine and 1-vinyl-3-methylimidazolium salt (e.g., Chloride salt) (referred to in the industry by the Cosmetic, Toiletry, and Fragrance Association, "CTFA". as Polyquaternium-16); copolymers of 1-vinyl-2-pyrrolidine and dimethylaminoethyl methacrylate (referred to in the industry by CTFA as Polyquaternium-11); cationic diallyl quaternary ammonium-containing polymers including, for example, dimethyldiallylammonium chloride homopolymer (referred to in the industry (CTFA) as Polyquaternium 6); mineral acid salts of amino-alkyl esters of homo-and co-polymers of unsaturated carboxylic acids having from 3 to 5 carbon atoms, as described in U.S. Patent 4,009,256; and cationic polyacrylamides as described in WO95/22311.

Other cationic deposition aids that can be used include cationic guar gum derivatives, such as guar hydroxypropyltrimonium chloride (Commercially available from Rhone Poulenc, ex Meyhall in their Jaguar trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the deposition aid is selected from the group comprising cationic polyacrylamides, and cationic guar derivatives. Particularly preferred deposition aids are Jaguar C13S with a cationic charge density of 0.8meq/g. Other particularly suitable materials include Jaguar C15, Jaguar C17 and Jaguar C16 and Jaguar C162.

The composition may further comprise from 0.1 to 5 % of a suspending agent. Examples are polyacrylic acids, cross linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearates, alkanolamides of fatty acids having from 16 to 22 carbon atoms and mixtures thereof. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol materials are available from Goodrich and Carbopol is a trade mark. A further suitable suspending agent is dihydrogenated tallow phthalic acid amide (available from Stepan Chemical Co. under the trademark Stepan TAB-2)

Suitable cross linked polymers of acrylic acid and acrylate esters are Pemulen TR1 or Pemulen TR2. A suitable heteropolysaccharide gum is xanthan gum, for example that available as Kelzan mu.

The hair treatment compositions of the invention are typically aqueous based. The compositions suitably comprise water in amount of from about 20 to about 99% by weight of the total composition.

The compositions of the invention are typically applied to the hair and/or scalp, left thereon for an appropriate period of time and then rinsed off with water.

Depending on the type of composition employed, one or more additional ingredients conventionally incorporated into hair treatment formulations may be included in the compositions of the invention. Such additional ingredients include opacifiers such as polyethylene glycol distearate and ethylene glycol stearates, polymer latices, additional antimicrobial agents such as ZnPTO and Octopirox, foam boosters, perfumes, colouring agents, preservatives, viscosity modifiers, proteins, polymers, buffering or pH adjusting agents, moisturising agents, herb or other plant extracts and other natural ingredients.

In a further aspect of the present invention there is provided a method of preparing an antimicrobial hair treatment composition which method comprises mixing together the components (a), (b) and (c) as are defined above, and adjusting the pH of the resultant composition to below 7, so that the antimicrobial compound of component (a) is present as insoluble particles in the final hair treatment composition.

Advantageously, by this pH adjustment, the antimicrobial compound can be made to crystallise out with a crystal morphology that lends its own pearlescence to the system. This reduces the requirement for added pearlescers, such as stearates.

A suitable acid for the pH adjustment step is citric acid.

The invention is further illustrated by way of the following non-limitative examples, in which all percentages are by weight based on total weight, unless otherwise specified.

### EXAMPLES

### Example 1 and Comparative Example A

The deposition of HP 101 (the monoethanolamine salt of the compound of formula (I) hereinabove described where R = octyl and X = oxygen, available commercially from Olin Corporation) from shampoo formulations was investigated in the following experiment.

### Protocol

Three shampoos were investigated:-

All shampoos were made from a common base, namely 14 % sodium lauryl ether sulphate (2EO), 2% cocamidopropylbetaine, 0.1 % JAGUAR C13S (guar hydroxypropyltrimonium chloride, ex Meyhall), and suspending, colouring and preserving agents. In addition, 5 % HP 101 was added to the test shampoos as follows:-

### Example 1 contained 5% HP101, with a shampoo pH of 4.1

### Comparative Example A contained 5% HP101, with a shampoo pH of 8.3

### Control contained no HP101, with a shampoo pH of 4.6.

Shampoo pHs were adjusted with HCl/NaOH where necessary.

### Visual Observations

| Shampoo | Appearance |
|---|---|
| Example 1 | Opaque, pale green |
| Comp.Ex.A | Transparent, green |
| Control | Transparent, colourless |

The above observations indicate that the HP101 is present in insoluble form in the shampoo of Example 1, and in soluble form in the shampoo of Comparative Example A.

### Deposition Testing

Deposition of HP 101 onto pigskin from each of the shampoos of Example 1, Comparative Example A and Control (no HP101) was measured using the following protocol:
1) Piglet skin was washed with ethanol and wiped dry using Kimwipe
2) 5 x 25mm diameter plastic cylinders were clamped onto the skin to provide a sealed surface area
3) The test shampoo was diluted 1:10 with water
4) 500µl of the diluted shampoo was applied to the test site on the skin and rubbed with a small test tube for 1 minute to simulate the washing action.
5) The test site was washed with 2 aliquots of 5ml of distilled water.
6) 1ml of ethanol was applied to the test site and rubbed for 30 seconds to recover deposited HP101 by extraction.
7) The HP101-containing ethanol solution was collected in a labelled sample vial
8) The procedure was repeated for other skin sites giving 5 replicates per shampoo, and a new piece of skin was used for each test shampoo
9) The HP101-containing ethanol samples were analysed by a colorimetric technique using a SHIMADZU UV-160A UV/VIS recording spectrophotometer.

After subtracting the measurements for the control shampoo, the detected deposition values were subjected to standard statistical analysis - namely the means and standard deviations were determined and the 95 % confidence interval measured as mean +/- (t times the standard deviation)/√n. Results are quoted in parts per million.

### Results

| Mean (corrected for control) | 95 % confidence limits |
|---|---|
| Example 1: 49.0 | 40.8, 57.3 |
| Comp.Ex.A: 18.8 | 12.9, 24.7 |
| Control: 0.0 | 0.0, 0.0 |

The results show that the low pH shampoo of the invention deposits significantly more HP101 than does the high pH shampoo of Comparative Example A under the wash-rinse regime used.

### Example 2

The following formulation illustrates an antimicrobial shampoo composition according to the invention.
14% Sodium lauryl ether sulphate 2EO
2% Cocamidopropyl betaine
0.5% Sodium benzoate
0.1% JAGUAR C13S (ex Meyhall)
0.75% HP101 (ex Olin)
0.4% CARBOPOL (ex Goodrich)

The pH of the composition was adjusted to 5.3, so that the HP101 was present in insoluble form.

## Claims

1. An antimicrobial hair treatment composition which is a shampoo composition comprising the following components:
(a) an antimicrobial compound of the formula: 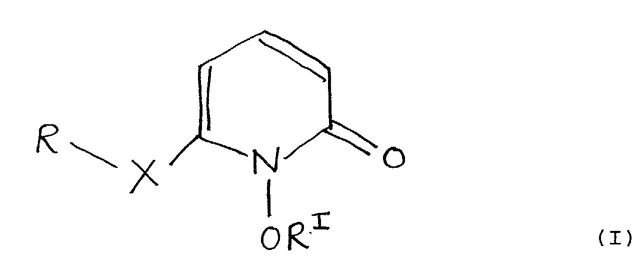 wherein X is an oxygen or sulphur moiety and R is a substituted or unsubstituted hydrocarbon radical having between 1 and 20 carbon atoms, with the proviso that R is other than chlorobenzyl, and R^{I} is hydrogen or a cation selected from the group consisting of alkali metals, alkaline earth metals, ammonium ion and organic ammonium ions, or a compound of the formula: 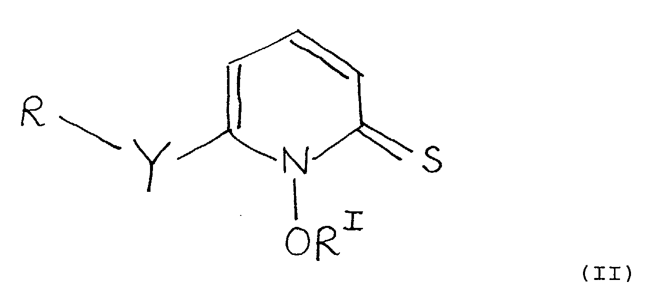 wherein Y is an oxygen, sulphur, or NR^{II} moiety wherein N is nitrogen and R and R^{II} are independently each a substituted or unsubstituted hydrocarbon radical having between 1 and 20 carbon atoms, or a compound of the formula: 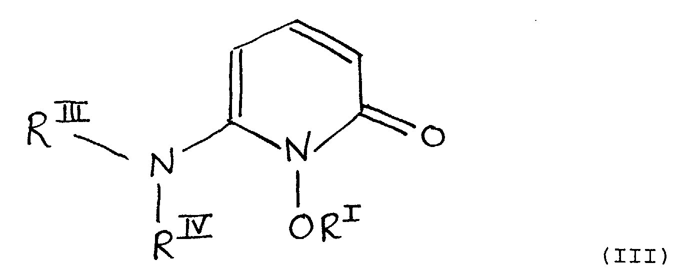 wherein R^{III} and R^{IV} are independently either hydrogen or a substituted or unsubstituted hydrocarbon radicals having between 1 and 20 carbon atoms, with the proviso that either R or R^{II} is a hydrocarbon radical;
(b) at least one shampoo surfactant; and
(c) a polymeric, water-soluble cationic deposition aid in which the pH of the hair treatment composition is less than 7, so that the antimicrobial compound is present as insoluble particles in the hair treatment composition.

2. A composition according to claim 1, in which the antimicrobial compound is present in the composition in an amount from about 0.1 to about 3% by weight.

3. A composition according to any preceding claim in which the deposition aid is a cationic derivative of guar gum or a cationic polyacrylamide.

4. A composition according to any preceding claim, in which the shampoo surfactant is selected from anionic, nonionic, amphoteric and zwitterionic surfactants, and mixtures thereof, in a total amount of from about 0.1 to about 50% by weight of the composition.

5. A composition according to any preceding claim, which further comprises a conditioning agent selected from volatile and non-volatile silicones.

6. A composition according to any preceding claim, which has a pH in the range from 4 to 6.

7. A method of preparing an antimicrobial hair treatment composition which comprises mixing together the components (a), (b) and (c) as are defined in Claim 1, and adjusting the pH of the resultant composition to below 7, so that the antimicrobial compound of component (a) is present as insoluble particles in the final hair treatment composition.
